# EUROPEAN PATENT APPLICATION

(11) **EP 1 286 165 A2**
(43) Date of publication of application: **26.02.2003**
(21) Application number: 02255380.4
(22) Date of filing: 01.08.2002
(51) Int. Cl.: G01N 33/74, G01N 33/566, A61K 38/00

(54) **Neuropeptide receptor and uses thereof**

(30) Priority: 15.08.2001 GB 0119920
(71) Applicant: Pfizer Limited, Sandwich, Kent CT13 9NJ (GB); PFIZER INC., New York, N.Y. 10017 (US)
(72) Inventor: Fidock, Mark David, c/o Pfizer Global Research, Sandwich, Kent CT13 9NJ (GB); Robas, Nicola Melanie, c/o Pfizer Global Research, Sandwich, Kent CT13 9NJ (GB)
(74) Representative: Hayles, James Richard

(57) **Abstract**

This invention relates to the identification of an orphan G-protein coupled receptor EBI2 (and variants thereof) as a receptor of neuropeptides of the CRF family, and the use of such peptides, e.g. urotensin I or sauvagine (and analogues or mimetics of either one) as modulators for EBI2. It also relates to screening methods to identify agonists and antagonists for this neuropeptide receptor.

SEQUENCE LISTING

<110> Pfizer Ltd. (GB)
      Pfizer Inc. (US, JP, EP except GB)

<120> Neuropeptide receptor and uses thereof

<130> PCS22032

<150> GB 0119920.7
<151> 2001-08-15

<160> 8

<170> FastSEQ for Windows Version 4.0

<210> 1
<211> 1086
<212> DNA
<213> Homo sapiens

<400> 1
atggatatac aaatggcaaa caattttact ccgccctctg caactcctca gggaaatgac 60
tgtgacctct atgcacatca cagcacggcc aggatagtaa tgcctctgca ttacagcctc 120
gtcttcatca ttgggctcgt gggaaactta ctagccttgg tcgtcattgt tcaaaacagg 180
aaaaaaatca actctaccac cctctattca acaaatttgg tgatttctga tatacttttt 240
accacggctt tgcctacacg aatagcctac tatgcaatgg gctttgactg gagaatcgga 300
gatgccttgt gtaggataac tgcgctagtg ttttacatca acacatatgc aggtgtgaac 360
tttatgacct gcctgagtat tgaccgcttc attgctgtgg tgcaccctct acgctacaac 420
aagataaaaa ggattgaaca tgcaaaaggc gtgtgcatat ttgtctggat tctagtattt 480
gctcagacac tcccactcct catcaaccct atgtcaaagc aggaggctga aaggattaca 540
tgcatggagt atccaaactt tgaagaaact aaatctcttc cctggattct gcttggggca 600
tgtttcatag gatatgtact tccacttata atcattctca tctgctattc tcagatctgc 660
tgcaaactct tcagaactgc caaacaaaac ccactcactg agaaatctgg tgtaaacaaa 720
aaggctctca acacaattat tcttattatt gttgtgtttg ttctctgttt cacaccttac 780
catgttgcaa ttattcaaca tatgattaag aagcttcgtt tctctaattt cctggaatgt 840
agccaaagac attcgttcca gatttctctg cactttacag tatgcctgat gaacttcaat 900
tgctgcatgg acccttttat ctacttcttt gcatgtaaag ggtataagag aaaggttatg 960
aggatgctga aacggcaagt cagtgtatcg atttctagtg ctgtgaagtc agcccctgaa 1020
gaaaattcac gtgaaatgac agaaacgcag atgatgatac attccaagtc ttcaaatgga 1080
aagtga 1086

<210> 2
<211> 361

<212> PRT
<213> Homo sapiens

<400> 2

<210> 3
<211> 27
<212> DNA
<213> Homo sapiens

<400> 3
accatggata tacaaatggc aaacaat                                          27

<210> 4
<211> 27
<212> DNA
<213> Homo sapiens

<400> 4
tcactttcca tttgaagact tggaatg                                          27

<210> 5
<211> 23
<212> DNA
<213> Homo sapiens

<400> 5
aggtgtgaac tttatgacct gcc                                              23

<210> 6
<211> 24
<212> DNA
<213> Homo sapiens

<400> 6
actgtaaagt gcagagaaat ctgg                                             24

<210> 7
<211> 18
<212> PRT
<213> Homo sapiens

<400> 7
Lys Gln Glu Ala Glu Arg Ile Thr Cys Met Glu Tyr Pro Asn Phe Glu
 1               5                  10                  15
Glu Thr

<210> 8
<211> 20
<212> PRT
<213> Homo sapiens

<400> 8
Lys Leu Phe Arg Thr Ala Lys Gln Asn Pro Leu Thr Glu Lys Ser Gly
1               5                   10                  15
Val Asn Lys Lys
            20

## Description

### Technical Field of the Invention

This invention relates to the identification of an orphan G-protein coupled receptor EBI2 (and variants thereof) as a receptor of neuropeptides of the CRF family, and the use of such peptides (and structurally related molecules) as modulators for EBI2. It also relates to screening methods to identify agonists and antagonists for this neuropeptide receptor.

### Background to the Invention

G-protein coupled receptors are a large superfamily of integral membrane proteins, involved in a broad range of signalling pathways. Most G protein-coupled receptors are characterised by 7 transmembrane-spanning helices, and are therefore also called 7-transmembrane receptors (7TMs). There are at least several hundred members of this family, which include receptors responding to a wide range of different stimuli, including peptides, biogenic amines, lipids, neurotransmitters, hormones, nucleotides, sugar-nucleotides, cytokines, etc. Structurally, the receptors of this family consist of an extracellular amino terminal domain, seven membrane-spanning hydrophobic regions, six loop regions, three of which are extracellular, the other three being intracellular, and an intracellular carboxy terminal domain. The hydrophobic regions show considerable homology between the different members of this family, whereas the loop regions, as well as the amino and carboxy terminal domains, are quite diverse, showing high homology only amongst closely related receptor subtypes.

Through analysis of sequence homology, this superfamily can be subdivided into families and subfamilies; for example, in general those receptors with peptide ligands show more sequence homology to other GPCRs which respond to peptide ligands than to those which respond to for example, bioactive amines or lipids etc.

The conserved structure and hydrophobicity profile of this receptor family has allowed the identification and cloning of many members of this family, both through cloning experiments, e.g. degenerate polymerase chain reaction or cDNA library screening with probes derived from GPCR sequences using low stringency, and through bioinformatic mining of sequence databases. For many of these receptors, it is yet unclear what their natural ligand and function are. These receptors are referred to as 'orphan receptors'. Through detailed analysis of their sequences and comparison with the sequences of GPCRs with known ligands, hypotheses can be generated as to the class of ligands that the orphan receptor may respond to.

However, these predictions are often tenuous, and it is still difficult to identify the ligand that such an orphan receptor may respond to. This is mainly because stimulation of GPCRs can lead to a multitude of different intracellular effects, which are mediated through the coupling to different G proteins. The G proteins in turn act on a whole range of signalling proteins, including phospholipase C, adenylate cyclase and ion channels, and thereby initiate a cascade of events in the cells.

G proteins are heterotrimeric complexes, containing an α-, and a βγ-subunit. At least 20 human genes are known to encode the GTP-binding α-subunits. The α-subunit family is divided into four subfamilies on the basis of their homology:
- The Gₛ family is typically able to stimulate adenylate cyclase upon agonist binding to the receptor coupling to it, and therefore lead to an increase in intracellular cyclic AMP.
- The G_{q} family, which includes G_{qα}, G_{11α}, G_{14α}, G_{16α}, lead to activation of phospholipase C.
- G₁₂ and G₁₃ appear to regulate classes of small molecular weight G proteins and Na⁺-H⁺ exchange.
- The G; family typically mediate inhibition of adenylate cyclase. More than half of the α-subunits are members of the G; family and include the ubiquitously expressed and nearly identical G_{i1α}, G_{i2α} and G_{i3α}, as well as several with a limited expression, such as G_{zα}, G_{oα} and transducin. Furthermore, there are at least 5 genes encoding the β-subunits and at least 11 genes encoding γ-subunits.

Briefly, initiation of signal transduction cascades involving G proteins requires the binding of an agonist ligand to a G protein-coupled receptor (GPCR). This results in the stabilisation of conformations of the GPCR that increase the rate of dissociation of the bound GDP from the nucleotide binding pocket of G_{α} subunit of a cognate G protein. Binding of GTP is thus allowed. With GTP bound, the G protein dissociates into GTP-G_{α} and G_{βγ} moieties and can hence regulate directly or otherwise the activity of several enzymes, which generate intracellular signalling molecules (e.g. cyclic AMP), or the probability of opening a range of ion channels (Gilman, A.G. (1987) Annu Rev. Biochem. 56, 614-649). Following G protein activation, hydrolysis of bound GTP by intrinsic GTPase activity terminates its ability to regulate its effectors and leads to α-GDP reassociation with βγ. The G protein therefore serves a dual role, as a relay transmitting the signal from the receptor to the effector, and as a clock that controls the duration of the signal. Therefore, depending on which G protein the GPCR couples to, very different intracellular effects can result, e.g. stimulation or inhibition of adenylate cyclase, leading to increased or decreased cyclic AMP concentrations in the cells, opening of ion channels, etc.

It is difficult to predict from the sequence of the GPCR which G protein it will couple to, and in some cases the resulting intracellular effects are difficult to measure. Furthermore, the usual host cells used for heterologous expression of cloned GPCRs only express certain G proteins; if the G protein that the orphan GPCR couples to is not expressed in these cells, the binding of an agonist to this receptor will not have a measurable effect, making it unlikely that one could find the agonist(s) for the GPCR in such a system.

It was therefore necessary to engineer specialised host cell lines, expressing various heterologous G proteins, to enable the establishment of host cell systems that can lead to measurable responses for many of the different GPCRs. There are also promiscuous G proteins, which appear to couple several classes of GPCRs to the phospholipase C pathway, such as G_{α15} or G_{α16} (Offermanns, S & Simon, M.I. (1995) J. Biol. chem. 270, 15175-15180), or chimeric G proteins designed to couple a large number of different GPCRs to the same pathway, e.g. phospholipase C (Milligan, G & Rees, S (1999) Trends in Pharmaceutical Sciences 20, 118-124). Host cell lines engineered to express one of these G proteins are more likely to give measurable responses to agonists for a variety of GPCRs than those expressing only their endogenous G_{α} subunits.

GPCRs are very important targets for pharmaceutical intervention, and many agonists and antagonists of the GPCRs of known functions are important for the treatment of diseases. Examples include but are not limited to, β₁-adrenergic receptor antagonists used for treating hypertension, angina and heart failure, dopamine D2 antagonists used for the treatment of schizophrenia, and β₂-adrenergic receptor agonists used for the treatment of asthma. Over the past 15 years, nearly 350 therapeutic agents targeting GPCRs have been introduced onto the market, and it is estimated that around 30% of clinically prescribed drugs function as either agonists or antagonists at GPCRs.

Therefore, it is likely that amongst the orphan receptors, there are further therapeutic targets which can play a key role in treating or preventing diseases or dysfunctions, including, but not limited to, pain, cancers, obesity, eating disorders, hypertension, hypotension, heart failure, incontinence, asthma, chronic bronchitis, angina, ulcers, psychotic and neurological disorders, viral infections including HIV-1 or HIV-2, other infections, inflammatory conditions, sexual dysfunction, urogenital disorders and many other disorders, diseases or dysfunctions. It is therefore clearly highly desirable to find the ligand and the physiological function for each GPCR.

EBI2 was first published by Birkenbach, M.P. et al (1993), J. Virol. 67, 2209-2220, and is also disclosed in patent application WO 94/12519 and patent US 5,744,301. Two transcripts, hypothesised to encode GPCRs because they showed some homology to GPCRs and showed 7 putative transmembrane regions, were found to be upregulated by EBV infection in B-lymphocytes, called EBI1 and EBI2.

EBI1 has since been identified as a chemokine receptor and renamed CCR7 (Yoshida, R. et al (1997) J. Biol. Chem. 272, 13803-13809). EBI1 and the ligands for EBI1, namely ELC (EBI1 ligand chemokine, Yoshida, R. et al (1997) J. Biol. Chem. 272, 13803-13809), also called CKβ-11 or macrophage inflammatory protein-3(β (Kim, C.H. et al (1998) J. Immunol. 160, 2418-2424), and secondary lymphoid tissue chemokine (Yoshida R. et al (1998) J. Biol. Chem. 273, 7118-7122), play roles in the migration and homing of normal lymphocytes as well as in the pathophysiology of lymphocytes. There are also reports linking EBI1 expression with the progression of adult T-cell leukemia (ATL) (Kohno T et al (2000) Japanese Journal of Cancer Research 91, 1103-1110). It was not unexpected that EBI1 is a chemokine receptor: it was most closely related to IL-8 receptors (Birkenbach M. et al (1993) J. Virol. 67, 2209-2220), and infection with EBV leads to the promotion of the production of factors such as IL-8, IL-10, TNF-α and TNF-β (Klein SC et al (1996) Leukemia Research 20, 633-636).

EBI2 showed much less homology to any known receptors at the time it was identified; Birkenbach et al ((1993) J. Virol. 67, 2209-2220) mention that it had some limited homology to thrombin receptors, even less homology to some other peptide receptors such as vasoactive intestinal polypeptide receptors, somatostatin receptors, angiotensin II receptors as well as the low affinity IL-8 receptor. But the ligand remained elusive, and very little is known about a function for EBI2 or what advantage it may have for EBV, as it is highly upregulated in B-lymphocytes upon EBV infection. There is a recent report that EBI2 is also amongst the genes differentially expressed in chronic lymphocytic leukemia (CLL) with 11q23 deletion; it appears that EBI2 is downregulated in Binet stages B and C (Aalto, Y. et al (2001) Leukemia 15, 1721-1728). Birkenbach et al ((1993) J. Virol. 67, 2209-2220) suggest that it is mainly expressed in B-lymphocytes and in lung, suggesting that it may be involved in B-lymphocyte function in some way, but no suggestion of its role in lung was made.

A Blast search against a non-redundant set of all known human GPCRs now reveals the top 6 hits as:
- P2Y5 receptor (SwissProt accession number P43657),
- CysLT1 receptor (GenBank accession number AF119711),
- orphan GPR17 (SwissProt accession Q13304),
- CysLT2 receptor (GenBank accession AB041644)
- P2Y9 receptor (SwissProt accession Q99677)
- CCR1 receptor (SwissProt accession P32246)

This would suggest that the ligand could be a molecule similar to a nucleotide or a leukotriene, or possibly another chemokine. The observation our invention is based on, namely that EBI2 is in fact a receptor for neuropeptides of the CRF family, such as urotensin I or sauvagine, is therefore highly surprising.

The identification of a new ligand, urocortin II (UcnII), for the CRF (corticotropin releasing factor) family of neuropeptides opens the possibility of the existence of additional receptors for this class of ligands (Reyes, T.M. et al (2001) Proc.Natl.Acad.Sci. 98 2843-48). UcnII has been demonstrated to bind selectively to CRF-R2; however, the observation that cellular activation by UcnII elicited through binding of the neuropeptide did not closely mimic the distribution of CRF-R2, thereby suggesting the possibility of additional receptors. UcnII has been shown to have an effect on inhibiting food intake without any effect on gross motor activity.

With EBI2 we have identified one such additional GPCR that responds to some peptides of the CRF-family of neuropeptides, e.g. urotensin I and sauvagine, and therefore we are calling this receptor now CRF-like receptor. Modulators of this receptor will be useful for treating, for example, depression, anxiety-related disorders e.g panic and generalised anxiety disorders, stress, Alzheimer's, stroke, inflammatory disorders e.g. arthritis and rheumatoid arthritis , eating disorders such as anorexia nervosa, and obesity.

### Aspects of the Invention

One aspect of the invention is the use of urotensin I or sauvagine or analogues or mimetics of either one as ligand for EBI2, preferably as modulator of EBI2, even more preferably as agonist of EBI2. In a preferred aspect of the invention, urotensin I or sauvagine or analogues or mimetics of either one are used to elicit a functional response on EBI2. A preferred aspect is the use of urotensin I or sauvagine as ligand for EBI2, preferably as modulator of EBI2, even more preferably as agonist of EBI2.

A further aspect of the invention is a method for identifying a compound that modulates a CRF-like peptide-mediated process, comprising a) contacting a sample of EBI2 with a CRF-like peptide such as urotensin I or sauvagine in the presence or absence of a test compound or test compounds, b) determining the biological effects thereof. In a typical assay, the test compound will bind, and have effects, at the same site on EBI2 as urotensin I or sauvagine normally bind, although the skilled practitioner will recognise that this need not always be so. The methodologies of the present invention may also be used to identify compounds acting at sites on EBI2 remote to the urotensin I or sauvagine binding site. In one aspect, the method comprises (a) contacting EBI2 with ligand (urotensin I or sauvagine or a functional analogue or mimetic of either one) in the presence of a test compound; and (b) measuring the amount of ligand bound to EBI2 in the absence of a test compound, such that if the amount of bound ligand measured in (a) and (b) is different, then a compound that modulates the binding of ligand to EBI2 is identified. In one embodiment, the EBI2 contacted in step (a) is on a cell surface. In another embodiment, the EBI2 is immobilised on a solid surface. In another embodiment, the solid surface is a microtiter dish. In yet another embodiment, the EBI2 is a membrane preparation used in suspension.

Therefore, one aspect of the invention is a method of screening for compounds that are ligands, preferably modulators, even more preferably agonists or antagonists, of EBI2, using urotensin I or sauvagine or an analogue or mimetic of either one as ligand.

A further aspect of the invention is a method of screening for compounds that are ligands, preferably modulators, more preferably agonists or antagonists of CRF-like receptors, including but not limited to the following steps:
(a) contacting a sample of EBI2 with a ligand selected from urotensin I or sauvagine or an analogue or mimetic of either one;
(b) contacting a similar sample of EBI2 with the same ligand as in step (a) and a test compound or mixture of test compounds; and
(c) comparing the results in (a) and (b) to determine whether the binding of the ligand is affected by the presence of the test compound or mixture of test compounds.

Preferably the method includes but is not limited to the following steps:
(a) contacting a sample of EBI2 with a ligand, selected from urotensin I or sauvagine or an analogue or mimetic of either one, with a detectable label attached, in the presence or absence of a test compound or mixture of test compounds;
(b) measuring the amount of label bound,
whereby a reduction of bound label in presence as compared to the amount of bound label in the absence of a test compound or mixture of test compounds indicates that the test compound or one or more test compounds present in the mixture of test compounds bind(s) to CRF-like receptor.

Another aspect of the invention is the use of urotensin I or sauvagine or an analogue or mimetic thereof to modulate, preferably agonise or antagonise, the receptor encoded by a nucleotide sequence comprising SEQ ID NO 1, or variants or homologues thereof.

A further aspect of the invention is the use of a CRF-like receptor in an assay wherein said receptor comprises the protein sequence of SEQ ID NO 2, or is a variant or homologue thereof.

Another aspect of the invention is a method of expressing a CRF-like receptor, comprising transferring a suitable expression vector comprising SEQ ID NO 1 or variants or homologues thereof, into suitable host cells, and culturing said host cells under conditions suitable for the expression of the receptor. Suitable host cells are bacteria such as *Escherichia coli*, yeasts such as *Saccharomyces cerevisiae* or *Pichia pastoris*, or preferably insect cells, or more preferably, mammalian cells, most preferably the host cells are human cells. A further aspect of the invention is a method of expressing CRF-like receptor, comprising upregulating the expression of EBI2 in a suitable cell. This can be achieved, for example, by infecting B-lymphocytes or B-lymphocyte-derived cell lines with Epstein Barr Virus (Birkenbach et al (1993) J. Virol. 67, 2209-2220), or by insertion of a strong promoter in front of the EBI2 gene, for example similar to the method used for the expression of erythropoietin in patent EP 411678, but other ways of upregulating EBI2 expression are envisaged. The sample of EBI2 in the above methods can therefore be derived from cells expressing EBI2 naturally, cells where the expression of EBI2 is upregulated by various means available in the art or developed in future, or from cells which have been transfected with an expression construct which contains a sequence comprising SEQ ID NO 1 or a variant or homologue thereof, or a sequence which encodes the protein sequence of SEQ ID NO 2.

A sample of EBI2 comprises cells prepared as described above, or cells naturally expressing EBI2, or membrane preparations prepared from any cells expressing EBI2, or EBI2 protein enriched or purified from such cells or membranes. The skilled person will be well aware of methods that can be used to enrich or purify EBI2, which include affinity chromatography, size exclusion chromatography, ion exchange chromatography and other methods suitable for the separation of protein from complex mixtures.

Yet a further aspect of the invention is a method for screening for agonists of CRF-like receptors, including, but not limited to, the steps:
(a) adding a test compound or a mixture of test compounds to suitable cells expressing EBI2,
(b) measuring whether a functional response is seen.

In a preferred aspect of the invention, the functional response is a transient rise in intracellular calcium concentration, measured by using fluorescent dyes such as Fluo-3 or Indo-1 or by other means known to the person skilled in the art; in another preferred aspect of the invention, the functional response is an increase in the rate of respiration of the cells as measured by an increase in the rate of acidification of the medium surrounding the cells as measured by microphysiometry. In yet another preferred aspect of the invention, the functional response is an increase or decrease in the cyclic AMP concentration in the cells, as measured e.g. by increased activity of a reporter gene product wherein the coding region of the reporter gene is functionally linked to a promoter comprising at least one cyclic AMP response element. Other methods to measure changes in cyclic AMP concentration in cells are well known to the person skilled in the art. In a further preferred aspect of the invention, cells are transfected with a plasmid or plasmids leading to co-expression of EBI2 and GFP-β-arrestin complex, and an agonist of the receptor is identified by observing clustering of fluorescence, i.e. GFP-β- arrestin complex, on the cell surface. Suitable cells expressing EBI2 are selected from cells naturally expressing EBI2, cells where the expression of EBI2 has been upregulated as described above, or cells where EBI2 has been expressed by transfection with an expression construct which contains a sequence comprising SEQ ID NO 1 or a variant or homologue thereof, or a sequence which encodes the protein sequence of SEQ ID NO 2.

The invention also includes a method of screening for antagonists of CRF-like receptors, including, but not limited to, the following steps:
(a) adding a test compound or a mixture of test compounds to suitable cells expressing EBI2,
(b) adding a ligand selected from urotensin I or sauvagine or an analogue or mimetic of either one, or an agonist as identified by the methods described above;
(c)measuring whether a functional response is seen, identifying antagonists as the test compound(s) which reduce the functional response to the ligand used in step (b).

Suitable cells expressing EBI2 are selected from cells naturally expressing EBI2, cells where the expression of EBI2 has been upregulated as described above, or cells where EBI2 has been expressed by transfection with an expression construct which contains a sequence comprising SEQ ID NO 1 or a variant or homologue thereof, or a sequence which encodes the protein sequence of SEQ ID NO 2. Examples of functional responses that can be used in this method are as described above.

Another aspect of the invention are compounds identified by any of the methods of the invention, and pharmaceutical compositions comprising a compound identified by any of the methods of the invention.

A further aspect of the invention is a method of preparing a pharmaceutical composition which comprises determining whether a compound is a CRF-like receptor agonist or antagonist using any one of the methods of the invention, and admixing said compound with a pharmaceutically acceptable carrier.

The invention is therefore, inter alia, directed to methods for screening for compounds that are useful in the treatment of neuropeptide-related disorders, preferably CRF-like peptide-related disorders, such as depression, anxiety-related disorders e.g. panic and generalised anxiety disorders, stress, Alzheimer's, stroke, inflammatory disorders e.g. arthritis and rheumatoid arthritis, eating disorders such as anorexia nervosa, and obesity.

The invention also provides assays to further characterise the biological activity of such compounds (for example agonists or antagonists), and their resultant utilities. Certain identified compounds will be useful in the treatment and prevention of disorders and conditions in which neuropeptides such as CRF-like peptides participate, such as, for example, depression, anxiety-related disorders e.g. panic and generalised anxiety disorders, stress, Alzheimer's, stroke, inflammatory disorders e.g. arthritis and rheumatoid arthritis, eating disorders such as anorexia nervosa, and obesity.

Another aspect of the invention is a method for detecting CRF-like peptide-related disorders in a mammal comprising measuring the level of EBI2 gene expression or of EBI2 receptor, in a patient sample, such that if the measured level differs from the level found in clinically normal individuals, then a CRF-like peptide/EBI2 related disorder is detected. Also provided are kits for measuring EBI2, e.g. comprising an antibody specifically recognising EBI2 (see below), or comprising the means to measure EBI2 mRNA, such as a probe hybridising to the EBI2 mRNA, or primers suitable for a PCR-based detection of the EBI2 mRNA or cDNA.

By way of example, levels of EBI2 can be detected by immunoassay like enzyme-linked immunosorbant assays (ELISAs), radioimmunoassays (RIA), immunocytochemistry, immunohistochemistry, Western blots, or other methods familiar to the skilled person. Levels of EBI2 RNA can be detected by hybridization assays (e.g. Northern blots, *in situ*-hybridization), and EBI2 activity can be assayed by measuring binding activities as described above *in vivo* or *in vitro.* Translocations, deletions, and point mutations in EBI2 nucleic acids can be detected by Southern blotting, FISH, RFLP analysis, SSCP, PCR using primers that preferably generate a fragment spanning at least most of the EBI2 gene, sequencing of EBI2 genomic DNA or cDNA obtained from the patient, etc.

Compounds that affect EBI2 gene activity, e.g. by affecting transcription, interfering with splicing events, or translation, can also be identified using the methods of the invention. It should also be noted that compounds that modulate signal transduction resulting from agonist binding to EBI2 can be detected by methods of the invention, and are within the scope of the invention.

For the sake of clarity, the term "CRF-like receptor" refers to a receptor for neuropeptides of the CRF family, i.e. CRF-like peptides such as urotensin I or sauvagine.

In this document the terms "homologue" or "variant" refer to an entity having a certain homology with the amino acid sequence and nucleotide sequence specified in SEQ ID NOs 1 and 2. A homologous sequence is taken to include an amino acid sequence which may be at least 75, 85, or 90% identical, preferably at least 95 or 98% identical to the specified sequence, or a nucleotide sequence which may be at least 75, 85 or 90% identical, more preferably at least 95 or 98% identical to the specified sequence. Such sequence homology/identity can be easily assessed by publicly or commercially available bioinformatics software, such as Blast2 (Altschul, S.F. et al (1997) Nucl. Acids Res. 25, 3389-3402), or programs included in the GCG software package (Devereux et al (1984) Nucl. Acids Res. 12, 387; Wisconsin Package Version 10, Genetics Computer Group (GCG, Madison, Wisconsin), such as Bestfit or Gap.

In this document, the term "analogue" relates to any substance which is generally similar in structure to a reference agent, e.g. urotensin I or sauvagine, and has generally similar biological effect, at least under one condition of assay.

As used herein, the term "mimetic" relates to any substance (which includes, but is not limited to, a peptide, polypeptide, antibody or other organic chemical) which has the same or similar qualitative activity or effect as a reference agent, e.g. urotensin I or sauvagine.

The term "modulator of a receptor" refers to any substance which binds to, and has an effect on, the respective receptor.

The term "agonist of a receptor" refers to a substance which can stimulate the respective receptor.

The term "antagonist of a receptor" refers to a substance which can inhibit the stimulation of the respective receptor by an agonist.

The term "functional response" refers to the reaction that, for example, stimulation of a receptor leads to in a cell. In the case of G-protein coupled receptors, this can include, for example, a change in the concentration of cyclic AMP, a transient rise in intracellular calcium concentration, or an opening of an ion channel.

The term "reporter gene" refers to a gene encoding a protein that can be conveniently measured. Preferably a gene used as reporter gene is not naturally expressed in the cells used. Examples include β-galactosidase, luciferase, chloramphenicol transferase (CAT) or β-lactamase. Typically the reporter gene is inserted behind a promoter or a response element or several response elements linked to a basic promoter, and upon a stimulatory signal, the reporter gene is translated, and the activity of the corresponding enzyme is increased, which can be easily measured.

The term "compound" refers to any chemical entity, including but not limited to a small organic molecule, a peptide, a protein, a modified protein such as a glycoprotein or a lipoprotein, antibodies or fragments thereof, a nucleic acid such as DNA or RNA or modified nucleic acids, such as oligonucleotides with a modified backbone.

### Further Details about the Invention

The methods of the invention may be used to identify compounds, such as small organic molecules or peptides or proteins, for example, that modulate the interaction of the CRF-like peptide such as urotensin I, sauvagine or analogues or mimetics of either one. The substances which may be screened in accordance with the invention therefore also include antibodies and fragments thereof. Peptidomimetic organic compounds that bind, for example, to the extra-cellular domain (ECD) of EBI2 and either inhibit the activity triggered by the natural ligand *(i.e.,* antagonists) or mimic the activity triggered by the natural ligand (*i.e.,* agonists), may also be screened. Additionally, organic compounds, peptides, antibodies or fragments thereof, to which the ECD (or a portion thereof) of EBI2 is covalently attached may also bind to and therefore "neutralize" the EBI2 ligand. Screening of such complex reagents is also within the practice of the invention.

Compounds that may be used for screening include, but are not limited to, peptides such as, for example, soluble peptides, including but not limited to members of random peptide libraries; (see, e.g., Lam et al. (1991) Nature 354, 82-84; Houghten et al. (1991) Nature 354, 84-86), and combinatorial chemistry-derived molecular library made of D- and/or L-configuration amino acids, phosphopeptides (including, but not limited to, members of random or partially degenerate, directed phosphopeptide libraries; see, e.g., Songyang et al. (1993) Cell 72, 767-778), antibodies (including, but not limited to, polyclonal, monoclonal, humanized, anti-idiotypic, chimeric or single chain antibodies, and Fab, F(ab')₂ and Fab expression library fragments, and epitope-binding fragments thereof), and small organic or inorganic molecules.

In one embodiment of the present invention, peptide libraries may be used as a source of test compounds that can be used to screen in the methods of the invention. Diversity libraries, such as random or combinatorial peptide or nonpeptide libraries can be screened for molecules that specifically bind to the EBI2 receptor. Many libraries are known in the art that can be used, e.g., chemically synthesized libraries, recombinant (e.g. phage display libraries), and *in vitro* translation-based libraries.

Examples of chemically synthesized libraries are described in Fodor et al. (1991) Science 251, 767-773; Houghten et al. (1991), Nature 354, 84-86; Lam et al. (1991), Nature 354, 82-84; Medynski (1994) Bio/Technology 12, 709-710; Gallop et al. (1994), J. Medicinal Chemistry 37, 1233-1251; Ohlmeyer et al. (1993), Proc. Natl. Acad. Sci. USA 90, 10922-10926; Erb et al. (1994), Proc. Natl. Acad. Sci. USA 91, 11422-11426; Houghten et al. (1992) Biotechniques 13, 412; Jayawickreme et al. (1994), Proc. Natl. Acad. Sci. USA 91, 1614-1618; Salmon et al. (1993) Proc. Natl. Acad. Sci. USA 90, 11708-11712; PCT Publication No. WO 93/20242; and Brenner and Lerner (1992) Proc. Natl. Acad. Sci. USA 89, 5381-5383.

Examples of phage display libraries are described in Scott & Smith (1990) Science 249:386-390; Devlin et al. (1990) Science 249, 404-406; Christian, et al. (1992), J. Mol. Biol. 227, 711-718; Lenstra (1992) J. Immunol. Meth. 152, 149-157; Kay et al. (1993) Gene 128, 59-65; and PCT Publication No. WO 94/18318 dated August 18, 1994.

By way of examples of nonpeptide libraries, a benzodiazepine library (see e.g. Bunin et al. (1994), Proc. Natl. Acad. Sci. USA 91, 4708-4712) can be adapted for use. Peptoid libraries (Simon et al. (1992) Proc. Natl. Acad. Sci. USA 89, 9367-9371) can also be used. Another example of a library that can be used, in which the amide functionalities in peptides have been permethylated to generate a chemically transformed combinatorial library, is described by Ostresh et al. (1994) Proc. Natl. Acad. Sci. USA 91, 11138-11142).

Screening the libraries can be accomplished by any of a variety of commonly known methods. See, e.g., the following references, which disclose screening of peptide libraries: Parmley & Smith (1989) Adv. Exp. Med. Biol. 251, 215-218; Scott & Smith (1990) Science 249, 386-390; Fowlkes et al. (1992) BioTechniques 13, 422-427; Oldenburg et al. (1992) Proc. Natl. Acad. Sci. USA 89, 5393-5397; Yu et al. (1994) Cell 76, 933-945; Staudt et al. (1988) Science 241, 577-580; Bock et al. (1992) Nature 355, 564-566; Tuerk et al. (1992) Proc. Natl. Acad. Sci. USA 89, 6988-6992; Ellington et al. (1992) Nature 355, 850-852; U.S. Patent No. 5,096,815, U.S. Patent No. 5,223,409, and U.S. Patent No. 5,198,346, all to Ladner et al.; Rebar & Pabo (1993) Science 263, 671-673; and PCT Publication No. WO 94/18318.

Compounds that can be tested and identified methods described herein can include, but are not limited to, compounds obtained from any commercial source, including Aldrich (1001 West St. Paul Ave., Milwaukee, WI 53233), Sigma Chemical (P.O. Box 14508, St. Louis, MO 63178), Fluka Chemie AG (Industriestrasse 25, CH-9471 Buchs, Switzerland (Fluka Chemical Corp. 980 South 2nd Street, Ronkonkoma, NY 11779)), Eastman Chemical Company, Fine Chemicals (P.O Box 431, Kingsport, TN 37662), Boehringer Mannheim GmbH (Sandhofer Strasse 116, D-68298 Mannheim), Takasago (4 Volvo Drive, Rockleigh, NJ 07647), SST Corporation (635 Brighton Road, Clifton, NJ 07012), Ferro (111 West Irene Road, Zachary, LA 70791), Riedel-deHaen Aktiengesellschaft (P.O. Box D-30918, Seelze, Germany), PPG Industries Inc., Fine Chemicals (One PPG Place, 34th Floor, Pittsburgh, PA 15272). Further any kind of natural products may be screened using the methods of the invention, including microbial, fungal, plant or animal extracts.

Furthermore, diversity libraries of test compounds, including small molecule test compounds, may be utilized. For example, libraries may be commercially obtained from Specs and BioSpecs B.V. (Rijswijk, The Netherlands), Chembridge Corporation (San Diego, CA), Contract Service Company (Dolgoprudny, Moscow Region, Russia), Comgenex USA Inc. (Princeton, NJ), Maybridge Chemicals Ltd. (Cornwall PL34 OHW, United Kingdom), and Asinex (Moscow, Russia).

Still further, combinatorial library methods known in the art, can be utilized, including, but not limited to: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) Anticancer Drug Des.12, 145). Combinatorial libraries of test compounds, including small molecule test compounds, can be utilized, and may, for example, be generated as disclosed in Eichler & Houghten (1995) Mol. Med. Today 1, 174-180; Dolle (1997) Mol. Divers. 2, 223-236; and Lam (1997) Anticancer Drug Des. 12, 145-167.

Examples of methods for the synthesis of molecular libraries can be found in the art, for example in: DeWitt et al. (1993) Proc. Natl. Acad. Sci. USA 90, 6909; Erb et al. (1994) Proc. Natl. Acad. Sci. USA 91, 11422; Zuckermann et al. (1994) J. Med. Chem. 37, 2678; Cho et al. (1993) Science 261, 1303; Carrell et al. (1994) Angew. Chem. Int. Ed. Engl. 33, 2059; Carell et al. (1994) Angew. Chem. Int. Ed. Engl. 33, 2061; and Gallop et al. (1994) J. Med. Chem. 37, 1233.

Libraries of compounds may be presented in solution (e.g. Houghten (1992) Bio/Techniques 13, 412-421), or on beads (Lam (1991) Nature 354:82-84), chips (Fodor (1993) Nature 364, 555-556), bacteria (U.S. Patent No. 5,223,409), spores (Patent Nos. US 5,571,698; US 5,403,484; and US 5,223,409), plasmids (Cull et al. (1992) Proc. Natl. Acad. Sci. USA 89, 1865-1869) or phage (Scott and Smith (1990) Science 249, 386-390; Devlin (1990) Science 249, 404-406; Cwirla et al. (1990) Proc. Natl. Acad. Sci. USA 87, 6378-6382; and Felici (1991) J. Mol. Biol. 222, 301-310).

Screening the libraries can be accomplished by any of a variety of commonly known methods. See for example the following references, which disclose screening of peptide libraries: Parmley & Smith (1989) Adv. Exp. Med. Biol. 251, 215-218; Scott & Smith (1990) Science 249, 386-390; Fowlkes et al. (1992) BioTechniques 13, 422-427; Oldenburg et al. (1992) Proc. Natl. Acad. Sci. USA 89, 5393-5397; Yu et al. (1994) Cell 76, 933-945; Staudt et al. (1988) Science 241, 577-580; Bock et al. (1992) Nature 355, 564-566; Tuerk et al. (1992) Proc. Natl. Acad. Sci. USA 89, 6988-6992; Ellington et al. (1992) Nature 355, 850-852; U.S. Patent No. 5,096,815, U.S. Patent No. 5,223,409, and U.S. Patent No. 5,198,346, all to Ladner et al., Rebar & Pabo (1993) Science 263, 671-673; and PCT Publication No. WO 94/18318.

Computer modeling and searching technologies permit identification of compounds, or the improvement of already identified compounds, that can modulate the interaction of urotensin I or sauvagine with EBI2. Having identified such a compound or composition, the active sites or regions are identified. Such active sites might typically be ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site (typcially a binding site) is determined. This can be done by known methods, including X-ray crystallography, which can determine a complete molecular structure. On the other hand, solid or liquid phase NMR can be used to determine certain intra-molecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active (binding) site, either experimentally, by modeling, or by a combination, candidate modulating compounds can be identified by searching databases containing compounds along with information on their molecular structure. Such a search seeks compounds having structures that match the determined active (binding) site structure and that interact with the groups defining the active site. Such a search can be manual, but is preferably computer assisted. These compounds found from this search are potential CRF-like receptor-modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active (binding) site structure of the compound to determine if an improved fit or interaction results. In this manner systematic variations in composition, such as by varying side groups, can be quickly evaluated to obtain modified modulating compounds or ligands of improved specificity or activity.

Further experimental and computer modeling methods useful to identify modulating compounds based upon identification of the active (binding) sites of either EBI2 or urotensin I, sauvagine, and analogs and mimetics of either one, will be apparent to those of skill in the art.

Examples of molecular modeling systems are the CHARMm and QUANTA programs (Polygen Corporation, Waltham, MA). CHARMm performs the energy minimization and molecular dynamics functions. QUANTA performs the construction, graphic modelling and analysis of molecular structure. QUANTA allows interactive construction, modification, visualization, and analysis of the behavior of molecules with each other.

A number of articles review computer modeling of drugs interactive with specific proteins, such as Rotivinen et al. (1988) Acta Pharmaceutical Fennica 97, 159-166; Ripka (1988) New Scientist 54-57; McKinaly and Rossmann (1989) Annu. Rev. Pharmacol. Toxiciol. 29, 111-122; Perry and Davies, OSAR: Quantitative Structure-Activity Relationships in Drug Design pp. 189-193 Alan R. Liss, Inc. (1989); Lewis and Dean (1989) Proc. R. Soc. Lond. 236, 125-140 and 141-162); and, with respect to a model receptor for nucleic acid components, Askew et al. (1989) J. Am. Chem. Soc. 111, 1082-1090. Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario). Although these are primarily designed for application to drugs specific to particular proteins, they can be adapted to design of drugs specific to regions of DNA or RNA, once that region is identified.

EBI2 protein, polypeptides and peptide fragments, mutated, truncated or deleted forms of the EBI2 and/or EBI2 fusion proteins can be prepared for a variety of uses, including but not limited to the generation of antibodies, as reagents in diagnostic assays, the identification of other cellular gene products involved in the regulation of neuropeptide-related disorders, as reagents in assays for screening for compounds that can be used as pharmaceutical reagents in the treatment of neuropeptide-related disorders. Additionally, based upon information gained from interacting EBI2 and urotensin I or sauvagine or other CRF-like peptides it may bind, peptide fragments of EBI2 can be derived which inhibit the normal binding of circulating neuropeptides to EBI2 receptors molecules, for *in vivo* therapeutic use.

EBI2 peptides, polypeptides, and fusion proteins can be prepared by recombinant DNA techniques. For example, nucleotide sequences encoding one or more of the four extracellular domains of the EBI2 receptor can be synthesized or cloned and ligated together to encode a soluble extracellular domain of EBI2. The DNA sequence encoding one or more of the four extracellular loops can be ligated together directly or via a linker oligonucleotide that encodes a peptide spacer. Such linkers may encode flexible, glycine-rich amino acid sequences thereby allowing the domains that are strung together to assume a conformation that can bind EBI2 ligands. Alternatively, nucleotide sequences encoding individual domains within the extracellular loops can be used to express EBI2 peptides.

A variety of host-expression vector systems may be utilized, to express nucleotide sequences encoding the appropriate regions of EBI2 to produce such polypeptides. Where the resulting peptide or polypeptide is a soluble derivative (e.g. peptides corresponding to the extracellular domain; truncated or deleted in which the transmembrane helices and/or cellular domains are deleted) the peptide or polypeptide can be recovered from the culture media. Where the polypeptide or protein is not secreted, the EBI2 product can be recovered from the host cell itself.

The host-expression vector systems also encompass engineered host cells that express EBI2 or functional equivalents *in situ*, i.e. anchored in the cell membrane. Purification or enrichment of EBI2 from such expression systems can be accomplished using appropriate detergents and lipid micelles and methods well known to those skilled in the art. However, such engineered host cells themselves may be used in situations where it is important not only to retain the structural and functional characteristics of EBI2, but to assess biological activity, e.g. in drug screening assays.

The host-expression vector systems that may be used for purposes of the invention include but are not limited to microorganisms such as bacteria (*e.g*., *E. coli*, *B. subtilis*) transformed with recombinant bacteriophage DNA, plasmid DNA or cosmid DNA expression vectors containing EBI2 nucleotide sequences; yeast (e.g. *Saccharomyces, Pichia*) transformed with recombinant yeast expression vectors containing EBI2 nucleotide sequences; insect cell systems infected with recombinant virus expression vectors (e.g. baculovirus) containing EBI2 sequences; plant cell systems infected with recombinant virus expression vectors (e.g. cauliflower mosaic virus, CaMV; tobacco mosaic virus, TMV) or transformed with recombinant plasmid expression vectors (e.g. Ti plasmid) containing EBI2 nucleotide sequences; or mammalian cell systems (e.g. COS, CHO, BHK, 293, 3T3) harboring recombinant expression constructs containing promoters derived from the genome of mammalian cells (e.g. metallothionein promoter) or from mammalian viruses (e.g. the adenovirus late promoter; the vaccinia virus 7.5K promoter).

In bacterial systems, a number of expression vectors may be advantageously selected depending upon the use intended for the EBI2 gene product being expressed. For example, when a large quantity of such a protein is to be produced, for the generation of pharmaceutical compositions of EBI2 protein or for raising antibodies to the EBI2 protein, for example, vectors which direct the expression of high levels of fusion protein products that are readily purified may be desirable. Such vectors include, but are not limited, to the *E. coli* expression vector pUR278 (Ruther et al. (1983) EMBO J. 2, 1791), in which the EBI2 coding sequence may be ligated individually into the vector in frame with the lacZ coding region so that a fusion protein is produced; pIN vectors (Inouye & Inouye (1985) Nucleic Acids Res. 13, 3101-3109; Van Heeke & Schuster (1989) J. Biol. Chem. 264, 5503-5509), and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al. (1991) Proc. Natl. Acad. Sci. USA 88, 8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺•nitriloacetic acid-agarose columns and histidine-tagged proteins are selectively eluted with imidazole-containing buffers.

In an insect system, *Autographa califomica* nuclear polyhedrosis virus (AcNPV) is used as a vector to express foreign genes. The virus grows in *Spodoptera frugiperda* cells. The EBI2 coding sequence may be cloned individually into non-essential regions (for example the polyhedrin gene) of the virus and placed under control of an AcNPV promoter (for example the polyhedrin promoter). Successful insertion of a EBI2 gene coding sequence will result in inactivation of the polyhedrin gene and production of non-occluded recombinant virus (i.e. virus lacking the proteinaceous coat coded for by the polyhedrin gene). The recombinant viruses are then used to infect cells in which the inserted gene is expressed (e.g. see Smith et al. (1983) J. Virol. 46, 584; Smith, U.S. Patent No. 4,215,051).

In mammalian host cells, a number of viral-based expression systems may be utilized. In cases where an adenovirus is used as an expression vector, the EBI2 nucleotide sequence of interest may be ligated to an adenovirus transcription/translation control complex, e.g. the late promoter and tripartite leader sequence. This chimeric gene may then be inserted in the adenovirus genome by *in vitro* or *in vivo* recombination. Insertion in a non-essential region of the viral genome (e.g. region E1 or E3) will result in a recombinant virus that is viable and capable of expressing the EBI2 gene product in infected hosts (see, e.g. Logan & Shenk (1984) Proc. Natl. Acad. Sci. USA 81, 3655-3659). Specific initiation signals may also be required for efficient translation of inserted EBI2 nucleotide sequences. These signals include the ATG initiation codon and adjacent sequences. In cases where an entire EBI2 gene or cDNA, including its own initiation codon and adjacent sequences, is inserted into the appropriate expression vector, no additional translational control signals may be needed. However, in cases where only a portion of the EBI2 coding sequence is inserted, exogenous translational control signals, including, perhaps, the ATG initiation codon, must be provided. Furthermore, the initiation codon must be in frame with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc (see Bittner et al. (1987) Methods in Enzymol. 153, 516-544).

In addition, a host cell strain may be chosen which modulates the expression of the inserted sequences, or modifies and processes the gene product in the specific fashion desired. Such modifications (e.g. glycosylation) and processing (e.g. cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. Accordingly, eukaryotic host cells which possess the cellular machinery for proper processing of the primary transcript, glycosylation, and phosphorylation of the gene product may be used. Such mammalian host cells include, but are not limited to, CHO, Vero, BHK, HeLa, COS, MDCK, HEK293, 3T3 and WI38 cell lines.

For long-term, high-yield production of recombinant proteins, stable expression is preferred. For example, cell lines which stably express the EBI2 sequences described above may be engineered. Rather than using expression vectors which contain viral origins of replication, host cells can be transformed with DNA controlled by appropriate expression control elements (e.g. promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the EBI2 gene product. Such engineered cell lines may be particularly useful in screening and evaluation of compounds that affect the endogenous activity of the EBI2 gene product.

A number of selection systems may be used, including but not limited to the herpes simplex virus thymidine kinase (Wigler et al. (1977) Cell 11, 223), hypoxanthine-guanine phosphoribosyltransferase (Szybalska & Szybalski (1962) Proc. Natl. Acad. Sci. USA 48, 2026), and adenine phosphoribosyltransferase (Lowy et al. (1980) Cell 22, 817) genes can be employed in tk⁻, hgprt⁻ or aprt⁻ cells, respectively. Also, antimetabolite resistance can be used as the basis of selection for the following genes: dhfr, which confers resistance to methotrexate (Wigler et al. (1980) Natl. Acad. Sci. USA 77, 3567; O'Hare, et al. (1981) Proc. Natl. Acad. Sci. USA 78, 1527); gpt, which confers resistance to mycophenolic acid (Mulligan & Berg (1981) Proc. Natl. Acad. Sci. USA 78, 2072); neo, which confers resistance to the aminoglycoside G-418 (Colberre-Garapin et al. (1981) J. Mol. Biol. 150:1); and hygro, which confers resistance to hygromycin (Santerre et al. (1984) Gene 30, 147).

EBI2 expression in mammalian cells can also be achieved by upregulating the expression of EBI2. This can be achieved, for example, by infection of B-lymphocytes or B-lymphocyte-derived cell lines with Epstein Barr Virus (see Birkenbach, M.P. et al (1993), J. Virol. 67, 2209-2220), or other substances may be identified that also upregulate EBI2 expression by using methods of the invention. Upregulation of EBI2 expression can also be achieved by recombinant means, for example as described in Patent EP 411,678 for human erythropoietin. Other methods are available, such as the system developed by Athersys and described, for example, in patent applications WO 99/15650 or WO 00/49162.

Antibodies that specifically recognize one or more epitopes of EBI2, or epitopes of conserved variants of EBI2, or peptide fragments of EBI2 are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of EBI2 in a biological sample and may, therefore, be utilized as part of a diagnostic or prognostic technique whereby patients may be tested for abnormal amounts of EBI2. Antibodies that specifically recognize mutant forms of EBI2, may be particularly useful as part of a diagnostic or prognostic technique. Such antibodies may also be utilized in conjunction with, for example, compound screening schemes, as described, above, for the evaluation of the effect of test compounds on expression and/or activity of the EBI2 gene product. Additionally, such antibodies can be used in conjunction with the gene therapy techniques, e.g. to evaluate the normal and/or engineered EBI2-expressing cells prior to their introduction into the patient. Such antibodies may additionally be used as a method for the inhibition of abnormal EBI2 activity. Thus, such antibodies may, therefore, be utilized as part of neuropeptide-related disorder treatment methods.

For the production of antibodies, various host animals may be immunized by injection with EBI2, a EBI2 peptide (e.g. one corresponding to extracellular loops or the extracellular domain), truncated EBI2 polypeptides (EBI2 in which one or more domains, e.g. the transmembrane domain or cellular domain, has been deleted), functional equivalents of EBI2 or mutants of EBI2. Such host animals may include but are not limited to rabbits, mice, hamsters and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum*.

Polyclonal antibodies are heterogeneous populations of antibody molecules derived from the sera of the immunized animals.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, ((1975) Nature 256, 495-497 and U.S. Patent No. 4,376,110), the human B-cell hybridoma technique (Kosbor et al. (1983) Immunology Today 4, 72; Cole et al. (1983) Proc. Natl. Acad. Sci. USA 80, 2026-2030), and the EBV-hybridoma technique (Cole et al. (1985) Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (Morrison et al. (1984) Proc. Natl. Acad. Sci., 81, 6851-6855; Neuberger et al. (1984) Nature, 312, 604-608; Takeda et al. (1985) Nature, 314, 452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region.

Alternatively, techniques described for the production of single chain antibodies (U.S. Patent 4,946,778; Bird (1988) Science 242, 423-426; Huston et al. (1988) Proc. Natl. Acad. Sci. USA 85, 5879-5883; and Ward et al. (1989) Nature 334, 544-546) can be adapted to produce single chain antibodies against EBI2 gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments or by papain digestion of antibody molecules. Alternatively, Fab expression libraries may be constructed (Huse et al. (1989) Science, 246, 1275-1281) to allow rapid and easy identification of monoclonal Fab fragments with the desired specificity.

Antibodies to EBI2 can, in turn, be utilized to generate anti-idiotype antibodies that "mimic" EBI2, using techniques well known to those skilled in the art (see, e.g. Greenspan & Bona (1993) FASEB J 7, 437-444; and Nissinoff (1991) J. Immunol. 147, 2429-2438). For example antibodies which bind to the EBI2 extracellular domain and competitively inhibit the binding of neuropeptides to EBI2 can be used to generate anti-idiotypes that "mimic" the extracellular domain and, therefore, bind and neutralize neuropeptides. Such neutralizing anti-idiotypes or Fab fragments of such anti-idiotypes can be used in therapeutic regimens to neutralize the native ligand and treat neuropeptide-related disorders, such as depression, anxiety-related disorders e.g panic and generalised anxiety disorders, stress, Alzheimer's, stroke, inflammatory disorders e.g. arthritis and rheumatoid arthritis , eating disorders such as anorexia nervosa, and obesity.

Alternatively, antibodies to EBI2 that can act as agonists of EBI2 activity can be generated. Such antibodies will bind to the EBI2 and activate the signal transducing activity of the receptor. In addition, antibodies that act as antagonist of EBI2 activity, i.e. inhibit the activation of EBI2 receptor would be particularly useful for treating neuropeptide- related disorders, such as depression, anxiety-related disorders e.g panic and generalised anxiety disorders, stress, Alzheimer's, stroke, inflammatory disorders e.g. arthritis and rheumatoid arthritis , eating disorders such as anorexia nervosa, and obesity.

Genetically engineered cells that express soluble EBI2 extracellular domains or fusion proteins e.g. fusion Ig molecules can be administered *in vivo* where they may function as "bioreactors" that deliver a supply of the soluble molecules. Such soluble EBI2 polypeptides and fusion proteins, when expressed at appropriate concentrations, should neutralize or "mop up" the native ligand for EBI2, and thus act as inhibitors of EBI2 activity and may therefore be used to treat neuropeptide-related disorders, such as depression, anxiety-related disorders e.g panic and generalised anxiety disorders, stress, Alzheimer's, stroke, inflammatory disorders e.g. arthritis and rheumatoid arthritis , eating disorders such as anorexia nervosa, and obesity.

The compounds of the invention, i.e. modulators of EBI2 receptor, can be used to treat conditions such as, for example, depression, anxiety-related disorders e.g panic and generalised anxiety disorders, stress, Alzheimer's, stroke, inflammatory disorders e.g. arthritis and rheumatoid arthritis , eating disorders such as anorexia nervosa, and obesity.

Toxicity and therapeutic efficacy of such compounds can be determined by standard pharmaceutical procedures in cell cultures or experimental animals, e.g. for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e. the concentration of the test compound which achieves a half-maximal inhibition of symptoms) as determined in cell culture. Such information can be used to more accurately determine useful doses in humans. Levels in plasma may be measured, for example, by high performance liquid chromatography.

Pharmaceutical compositions for use in accordance with the present invention may be formulated in conventional manner using one or more physiologically acceptable carriers or excipients.

Thus, the compounds and their physiologically acceptable salts and solvates may be formulated for administration by inhalation or insufflation (either through the mouth or the nose) or oral, buccal, parenteral or rectal administration.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavoring, coloring and sweetening agents as appropriate.

Preparations for oral administration may be suitably formulated to, give controlled release of the active compound.

For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, e.g. by bolus injection or continuous infusion. Formulations for injection may be presented in unit dosage form, e.g. in ampules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g. sterile pyrogen-free water, before use.

The compounds may also be formulated in rectal compositions such as suppositories or retention enemas, e.g. containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### Examples

The examples below are carried out using standard laboratory procedures, well known to a person skilled in the art, but reference may be made in particular to Sambrook *et al*., Molecular Cloning, A Laboratory Manual (2001) and Ausubel *et al*., Short Protocols in Molecular Biology (1999) 4^{th} Ed, John Wiley & Sons, Inc. PCR is described in US-A-4683195, US-A-4800195 and US-A-4965188.

The examples are intended to illustrate but not limit the invention. While they are typical of the methods that might be used, other procedures known to those skilled in the art may also be used. References are made to the following sequence listings and figures:
SEQ ID NO: 1: cDNA sequence of EBI2.
SEQ ID NO: 2: Peptide sequence of EBI2.
SEQ ID NO: 3: Forward primer for cloning EBI2 cDNA
SEQ ID NO: 4: Reverse primer for cloning EBI2 cDNA
SEQ ID NO: 5: Forward primer for tissue distribution studies of EBI2
SEQ ID NO: 6: Reverse primer for tissue distribution studies of EBI2
SEQ ID NOs: 7 & 8: Peptides from EBI2 used for production of antibodies recognising EBI2 for immunohistochemistry studies

Figure 1: Response of EBI2-expressing cells to urotensin I.
Figure 2: Clustering of GFP-β-arrestin on the surface of HEK293 cells expressing EBI2 in response to urotensin I.

### Example 1: Bioinformatics analysis of the EBI2 sequence

### (a) Blast against protein databases

The sequence of EBI2 was searched against Swissprot using the BLAST algorithm (Basic Local Alignment Search Tool (Altschul SF (1993) J.Mol. Evol. 36, 290-300; Altschul, SF et al (1990) J. Mol. Biol. 215, 403-410; Altschul SF et al (1997) Nucl. Acids Res. 25, 3389-3402)) to identify the closest protein match. In this case the top hit was to: P32250 Chicken P2Y5 receptor.

These results confirm that EBI2 is a member of the GPCR family.

### (b) BLAST search against a non-redundant human GPCR database

The EBI2 sequence was searched against a non-redundant human GPCR database comprising mainly sequences from Genbank and the Derwent Geneseq database (containing sequences from patent applications/patents) in order to identify the class of agonist for this receptor. The top ten hits are shown below; these hits all have similar probability scores making the order of the hits arbitrary in real terms.

| | | | |
|---|---|---|---|
| P43657 (P2Y5_HUMAN) | P2Y purinoceptor 5 (P2Y5) | 173 | 4e-44 |
| AF133266 | Cysteinyl leukotriene receptor (CYSLT1) | 173 | 5e-44 |
| Q13304 (GPRH_HUMAN) | G protein-coupled receptor GPR17 | 172 | 6e-44 |
| AB038269 | Cysteinyl leukotriene receptor CysLT2 | 169 | 4e-43 |
| Q99677 (P2Y9_HUMAN) | P2Y purinoceptor 9 (P2Y9) | 168 | 9e-43 |
| P32246 (CKR1_HUMAN) | C-C chemokine receptor type 1 (C-C CK... | 166 | 4e-42 |
| P47900 | P2Y purinoceptor 1 (P2Y1) | 154 | 2e-38 |
| 000254 | Proteinase activated receptor 3 (PAR-3) | 153 | 3e-38 |
| P41231 | P2U purinoceptor 1 (P2U1) | 153 | 5e-38 |
| Q9UPC5 | G-protein coupled receptor GPR34 | 150 | 4e-37 |

### (c) Hidden Markov Models

The profile of a family of sequences can be modelled in a probabilistic way using hidden Markov models (HMMs) (Durbin, R., Eddy, S., Krogh, A. and Mitchison, G. (1998), Biological Sequence Analysis: Probabilistic models of proteins and nucleic acids; Cambridge University Press, Cambridge, UK.; Eddy, S.R. (1998) Bioinformatics 14, 755-763). HMMs were originally developed for use in speech recognition and have since been used for a range of signal processing applications. A signal is modelled as a sequence of elements from a finite set. In speech recognition this set consists of the sounds that make up the language and the aim is to deduce what words the sounds represent. A protein sequence from a particular family can be thought of in the same way. The sequence is a series of elements from the set of 20 amino acids and the requirement is to determine what protein family the sequence could represent.

Profile HMMs can be constructed from a multiple sequence alignment of known family members. Each position in the alignment corresponds to a state, where states are "match", "insert" or "delete". The whole alignment can therefore be thought of as a linear chain, or path, of interconnecting states connected by transition probabilities. These states "emit" amino acid residues with different probabilities (based on the range of residues seen in the alignment together with substitution data and "pseudocount" methods - to correct for limited data in the initial alignment). The probability of a particular sequence matching the model can be calculated as a combination of the transition probabilities between states and the emission probabilities for that amino acid sequence.

Once a good alignment of known family members has been obtained, HMMs can be constructed with little manual intervention (compared with profiles) and are a more sensitive method for detecting remote homologues. The Pfam database is a library of profile HMMs for many protein families (Bateman, A. et al (1999) Nucl. Acids Res. 27, 260-262). It contains models constructed from carefully edited seed alignments and models automatically generated by an iterative procedure. This complete automation of the process has the disadvantage that any errors that are made will be rapidly propagated.

For the present analysis, profile HMMs were constructed for every major GPCR subfamily by assembling all known members and aligning them with CLUSTAL. Each subfamily model was incorporated into a database and the novel GPCRs searched with the entire database to identify the best match.

The EBI2 sequence was screened against models representing all GPCR sub-families including a CysLt1/Lt2 model (cysLeuko), a general leukotriene model (i.e. including the two LTB receptors) and model representing the incorrectly annotated P2Y5/P2Y9/P2Y10 genes (orphanP2Y). The results shown below show a much more significant hit to the CysLT model than to any others, including the model containing P2Y5/P2Y9.

| | cysLeuko | leukotriene | purinoceptor | oxcChemo | ccChemokirie | orphanP2Y |
|---|---|---|---|---|---|---|
| Probability score (lowest = most significant) | 1.40E-36 | 3.50E-23 | 1.30E-17 | 2.50E-14 | 2.80E-14 | 6.40E-14 |

The profile from the Hidden Markov model analysis indicates that this receptor is most closely related to a GPCR binding cysteinyl leukotrienes.

### Example 2: Tissue distribution of EBI2

### (1) EBI2 mRNA

(a) A gene expression microarray, which, amongst 6800 human cDNAs, also contains EBI2, was used in a number of experiments, hybridising labelled probes derived from mRNA or total RNA from a variety of tissues and cells. Significant expression of this sequence was only found in the haemic/immune system and lung.
(b) By searching expressed sequence tag (EST) databases, EBI2 cDNA clones were identified in libraries from a wide variety of differently treated peripheral blood mononuclear cells, such as lymphocytes, eosinophils, macrophages, monocytes, dendritic cells, leukocytes, as well as lymph node, tonsil, and spleen. It was also found in various lung cDNA libraries. ESTs from this cDNA sequence were also found in various libraries from other tissues, albeit at much lower frequency.
(c) PCR using cDNA from various tissues is a more sensitive method to determine in which tissues the EBI2 transcript can be found. PCR was performed on 10ng of cDNA, using Clontech's "Multiple tissue panels" (Cat# K1420-1, K1421-1). The primers used were as follows:

Forward primer: 5'-AGGTGTGAACTTTATGACCTGCC-3' (SEQ ID NO: 5)

Reverse primer:  5'-ACTGTAAAGTGCAGAGAAATCTGG-3'  (SEQ ID NO: 6)

The PCR mixes were prepared and heated for 1 minute to 94°C, and then 40 cycles of 30 seconds at 94°C, 30 seconds at 58°C and 1 minute at 94°C were carried out. The results are summarised in the table below:

| **Tissue** | **Expression level** |
|---|---|
| Testis | ++ |
| Pancreas | ++ |
| Spleen | +++ |
| Ovary | ++ |
| Placenta | + |
| Prostate | ++ |
| Colon | ++ |
| Kidney | ++ |
| Skeletal muscle | - |
| Brain | ++ |
| Heart | + |
| Liver | ++ |

### (2) EBI2 Protein

The antibody production and immunohistochemistry were carried out in collaboration with LifeSpan BioSciences Inc, Seattle, USA. Two rabbit polyclonal antibodies, raised against peptide sequences from EBI2, shown below as SEQ ID NOs 7 and 8, were used in immunohistochemical studies to identify tissues and cell types where the EBI2 protein is expressed. The staining pattern produced with these antibodies was generally consistent with the expression pattern of EBI2 reported in the literature.

The peptide sequences used were:

SEQ ID NO 7: KQEAERITCMEYPNFEET

SEQ ID NO 8: KLFRTAKQNPLTEKSGVNKK

### Staining with antibody 1451-173 (produced against the peptide shown as SEQ ID NO 7)

In the central nervous system, neurons stained focally in all tissues, with more intense staining apparent within the amygdala, caudate and medulla (vagal, trigeminal, and arcuate nuclei).

In peripheral tissues, epithelium and lining cells stained variably with more intense staining detected in the urothelium of the bladder, lobular and ductal epithelium of the breast, focal absorptive and mucinous epithelium of the colon, bronchial respiratory epithelium, prostatic glandular and ductal epithelium, skin epidermis and adnexal glands, gastric mucosal chief cells, zona glomerulosa of the adrenal and secretory endometrium of the uterus. There was focally strong staining of hepatocytes and skeletal myocytes. Smooth muscle was stained variably.

Among malignancies, all samples tested showed at least focal staining, with the exception of small cell carcinoma of the lung. It is noteworthy that in the case of colon adenocarcinoma, the tumor stained uniformly while nearby benign glandular tissue was only rarely blush positive.

Among samples of non-neoplastic disease, there was increased staining of myointimal cells in atherosclerosis. Subsets of myocytes stained more intensely in myocardial infarct and heart failure. No significant differences in staining compared to normal tissue counterparts in samples of Alzheimer's disease, glioblastoma multiforme, brain infarct, Parkinson's disease, ulcerative colitis, Crohn's disease, diabetic heart and kidney, hypertensive kidney, asthma, bronchitis, pneumonia and emphysema of the lung, allergic rhinitis of the nasal mucosa, and benign prostatic hyperplasia.

### Staining with antibody 1451-222 (produced against the peptide shown as SEQ ID NO 8)

The staining pattern produced with this antibody was generally concordant with that of antibody 1451-173, and was consistent with the expression pattern of this GPCR reported in the literature. However, staining of plasma cells and other B lymphocytes, and Purkinje cells in the cerebellum was not seen with antibody 1451-173.

In the central nervous system, neurons showed focal staining in amygdala, caudate, cerebellum, cerebral cortex, hippocampus, hypothalamus, medulla, pituitary, putamen, substantia nigra, and thalamus. However, plasma cells, B lymphocytes, Purkinje cells in the cerebellum, cerebral cortex, dorsomedial and ventromedial nuclei of the hypothalamus, mammillary body nuclei, hypoglossal and arcuate nuclei of the medulla, anterior pituitary and posterior pituitary, and substantia nigra showed particularly intense staining. The adjacent ependyma and choroid plexus lining cells were generally positive. The staining intensity of the arcuate nucleus of medulla exceeded other neurons in the central nervous system.

In peripheral tissues, epithelium lining cells stained variably in transitional urothelium of the bladder, lobular and ductal epithelium of the breast, absorptive and mucous epithelium of the colon, bronchial respiratory epithelium, prostatic and ductal epithelium, skin epidermis and adnexal glands, and gastric mucosal chief cells. Of special note was the intense staining of hepatocytes and skeletal myocytes. Smooth muscle stained variably.

All malignancies tested showed at least focal staining, except for small cell carcinoma of the lung. It is noteworthy that in the sample of colon adenocarcinoma, the tumor was uniformly stained, while nearby benign glandular tissue only showed rare blush positivity.

Among non-neoplastic disease, increased staining was noted compared to normal tissue samples in myointimal cells in atherosclerosis, and myocytes near an area of heart infarct. Less intense staining was detected in respiratory epithelial cells in samples of lung from patients with bronchitis, asthma, emphysema and pneumonia and in surface epithelial cells in ulcerative colitis and Crohn's disease. No change in staining was noted in samples of brain from patients with Parkinson's disease, infarct, glioblastoma multiforme and Alzheimer's disease, samples of diabetic or hypertensive kidney, or diabetic heart

### Example 3: Cloning and transient expression of EBI2 in mammalian cell lines

The cDNA encoding EBI2 was obtained by polymerase chain reaction (PCR) of cDNA from peripheral blood leukocytes (PBL; Clontech). The PCR primers were designed around the putative ATG start codon and the stop codon, leading to the amplification of the coding sequence only, eliminating any 5' or 3' untranslated regions. The primers used are as shown in SEQ ID NOs 3 and 4:

EBI2-1: 5'-ACC ATG GAT ATA CAA ATG GCA AAC AAT -3' (SEQ ID NO: 3)

EBI2-2: 5'-TCA CTT TCC ATT TGA AGA CTT GGA ATG-3' (SEQ ID NO: 4)

The PCR mix was assembled as follows:

| | |
|---|---|
| EBI2 primers | 1µl (1µM final concentration) |
| PBL cDNA | 2µl |
| dNTPs (from Elongase kit) | 1µl |
| Elongase Polymerase (LTI Inc) | 1µl |
| Buffer B (from Elongase kit) | 10µl |
| DMSO | 5µl |
| dH₂O | 30µl |

The PCR cycles were the following: 35 cycles of denaturing at 94°C for 1min, annealing at 55°C for 1.5 mins, and extension at 68°C for 3 mins; after the last cycle, the temperature was held at 68°C for 10 mins, followed by cooling the reaction down to 4°C.

The PCR product was gel-extracted using the QIAgen gel extraction kit, according to the manufacturer's instructions. The resultant product was TA cloned (Invitrogen TA cloning methodology) into the vector pcDNA3.1/V5-His-TOPO (Invitrogen), according to the manufacturer's instructions. The resulting insert was subsequently sequence-verified on both strands using ABI DNA sequencing methodology as per the manufacturer's protocol. The PCR product was then inserted into a suitable mammalian cell expression vector, e.g. into pcDNA3.1/V5-His-TOPO (Invitrogen), using the manufacturer's protocols. The cDNA insert was excised from the pcDNA3.1/V5-His-TOPO vector, e.g. using restriction sites *Kpn*I and *Not*I, and ligated into a different expression vector, e.g. pcDNA4HISmaxB digested with the same enzymes, using standard molecular biology techniques (e.g. according to Sambrook, *et al*., eds. (1989) Molecular Cloning: A Laboratory Manual, Cold Spring Harbor Laboratory Press, New York, NY, USA) and using T4 DNA ligase to join the fragments. The vector pcDNA4HISmaxB is used as it contains elements that upregulate the level of gene transcription over standard pcDNA3.1 vectors. 5 x 10⁶ cells of a suitable host cell line, chosen to express a low number of endogenous receptors (e.g. HEK293 or CHO-K1) and engineered to stably express certain G_{α} proteins (e.g. mouse G_{α15} (Wilkie, T.M. et al (1991) Proc. Natl. Acad. Sci. 88, 10049-10053), a chimeric G_{qi5}/G_{qo5} α-subunit, or human G_{α16} (Amatruda, TT 3^{rd} et al (1991) Proc. Natl. Acad. Sci. 88, 5587-5591)), were transiently transfected with 7.5 µg of cDNA encoding EBI2 in a suitable mammalian cell expression vector, or vector alone, using Lipofectamine Plus® reagent (Gibco BRL) as per the manufacturer's protocol. If the cells were intended for a ligand binding assay or microphysiometry, they were incubated for 24-72 hours post transfection, and then harvested. If a microtitre plate-based assay was intended, the cells were detached from the flask using Trypsin/EDTA solution (LTI) 24 hours post transfection, and seeded into e.g. a black sided, Poly-D-lysine-treated, 96-well plate (Becton Dickinson) at 5 x 10⁴ cells/well density. The plates were left overnight to allow the cells to adhere to the bottom of the wells.

### Example 4: Engineering of stable cell lines expressing high levels of EBI2

A suitable host cell line, e.g. HEK293 cells or CHO cells (potentially engineered to express a desired G protein), was transfected as described in Example 3, with a suitable mammalian cell expression vector containing the cDNA (preferably without any 5' or 3' untranslated regions) encoding EBI2, and containing a selectable marker, e.g. a neomycin resistance gene. Following transfection, selection pressure was applied, e.g. by adding 400-800 µg/ml G418 to the growth medium and thereby killing all cells which have not taken up the vector which contains the neomycin resistance gene. After about 3-4 weeks of selection, individual clones were picked and expanded for further analysis.

An alternative strategy for making stable cell lines uses the Flp-In system (Invitrogen). The cDNA was cloned in to a vector containing a recombination target sites (FRT). The recombinant expression vector containing the cDNA was introduced in to the modified Flp-In cell line using standard techniques (lipofectamine or similar). The modified cell line already contains a recombination target site in the genome and on introduction of the second FRT contained within the expression vector homologous recombination occurs mediated by Flp recombinase; positive clones are selected with hygromycin. This resulted in stable integration of the expression construct at a single site within the genome and eliminated the need for clonal selection. The stable cell line was then expanded for further analysis, the whole process taking roughly 4 weeks.

The individual clones can be analysed e.g. by Northern blot, using a labelled probe designed from the EBI2 cDNA sequence or by the use of RT-PCR using the oligonucleotides as described above.

### Example 5: Ligand binding assays

Cells expressing EBI2, either 24-72 hours after transient transfection as described in Example 3, or engineered as described in Example 4, were harvested by scraping, resuspended in 20 ml of ice-cold assay buffer (50 mM Tris-HCl pH 7.4), homogenised, and the resulting suspension was centrifuged at 20,000g, 4°C for 30 minutes. The supernatant was decanted, the pellet resuspended in 3 ml of assay buffer and re-homogenised (50 mM Tris-HCl pH7.4). The protein concentration was determined via Bradford's assay (Biorad), according to the manufacturer's recommendations.

Aliquots of this membrane preparation containing 200 µg protein were then incubated with various potential ligands, radiolabeled to high specific activity, for about 2 hrs at room temperature or at 30°C (the optimal conditions, ion concentrations, incubation time and temperature need to be determined for each ligand). To terminate incubations, samples were rapidly filtered using the Brandell cell harvester onto Wallac Filtermats (Perkin Elmer) (which had been previously soaked (for 1h) in a 0.3% (v/v) solution of PEI (polyethylenimine; Sigma) in assay buffer to reduce Filtermat binding). Immediately, the Filtermat/wells were washed four times in rapid succession with 2 ml of assay buffer per well. Filtermats were dried using a microwave oven, and Meltilex scintillant (Perkin Elmer) was melted onto the Filtermats using the Wallac Meltilex heat sealer. The bound radioactivity on the Filtermats was determined using the Wallac betaplate scintillation counter.

The specific binding was defined as the difference between total radioactivity bound minus the radioactivity measured in the presence of an excess of unlabelled ligand. Mock-transfected cells were also measured to assess whether the host cells express receptors for the ligands used endogenously.

Alternatively, a scintillation proximity assay can be used. Purified or partially purified membranes of EBI2 expressing cells are coated onto the surface of a scintillant-loaded solid phase, e.g. beads, and the solid phase is treated with a blocking agent such as albumin or serum. Radiolabelled ligand (e.g. ³³P-labelled or ³H-labelled urotensin I or sauvagine or analogue or mimetic thereof) is then mixed with the EBI2-coated beads, under conditions that would allow specific binding to occur, in the presence or absence of test compounds. The binding of the labelled ligand to the EBI2 brings the radioactivity in close proximity to the scintillant-loaded surface (e.g. beads), which allows the scintillant to emit light which is detectable with a scintillation counter. A test compound that competes or otherwise interfers with the binding of the ligand to EBI2 will lead to a reduction of light emission from the respective sample as compared to the sample without a test compound added. Many variations of this basic assay format are available and known to the skilled person.

### Example 6: Functional assay measuring intracellular calcium mobilisation

Fluorescence Imaging Plate Reader (FLIPR®) technology was employed as a means to detect activation of EBI2 by various potential GPCR agonists.

Cells were transfected as described in Example 3. 24 hrs post-transfection, the cells were detached from the flask using Trypsin/EDTA solution (LTI) and seeded into a black sided, Poly-D-lysine-treated, 96-well plate (Becton Dickinson) at 5 x 10⁴ cells/well density. The plates were left overnight to allow the cells to adhere to the bottom of the wells. The medium was removed from the cells and replaced with 100 µl warm (37°C) dye loading solution (50 µg Fluo3 (Molecular Probes) in 20 µl DMSO + 20% pluronic acid in DMSO, added to 11 ml Dulbecco's Modified Eagles Medium containing 1x Probenecid (100x Probenecid - 0.71 g Probenecid was dissolved in 5 ml 1M NaOH and 5 ml Dulbeccos' Phosphate Buffered Saline (PBS), per plate; Probenecid (Molecular Probes) inhibits activity of the anion transport protein, thus improving dye loading). The plates were then incubated for 1 hr at 37°C. Plates were subsequently washed with 250 µl of wash buffer per well (5 ml 100x Probenecid stock + 495 ml PBS, pH 7.4) 4 times. The plates were returned to the 37°C/5%CO₂ incubator for 30 mins prior to processing within the FLIPR® instrument. The FLIPR® processing involved reading the fluorescence for all samples for 2 minutes; during this time the fluorescence baseline was determined for 10 seconds. The desired amount of compounds (i.e. potential GPCR agonists) was then automatically transferred to the wells and the fluorescence was continuously monitored for the remainder of the time. All compounds were diluted in wash buffer.

Compounds capable of acting as agonists for the receptor were identified by them causing a transient rise in fluorescence, and therefore in intracellular calcium, in the cells.

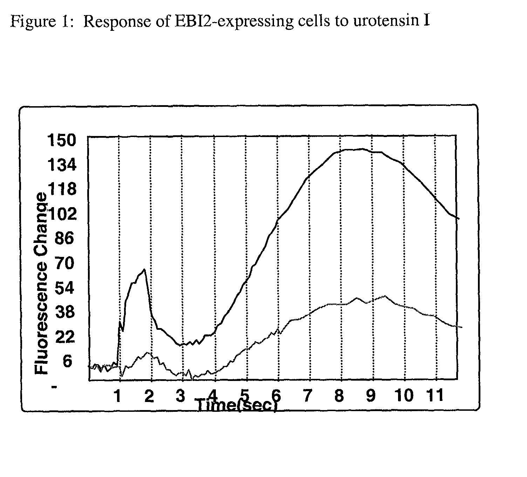

The response of EBI2-expressing cells to urotensin I is shown in Figure 1 (black line), showing fluorescence change over time, as compared to mock-transfected cells (grey line).

### Example 7: Functional assay using microphysiometry

Agonism at most receptors will lead to the cells requiring extra energy to respond to the stimulus. In order to generate the energy, the metabolic activity will increase, leading to a small but measurable acidification of the surrounding medium. This small pH change can be measured using the Cytosensor microphysiometer developed by Molecular Devices Corporation.

Cells expressing EBI2, produced as described in Example 3 or 4, were harvested gently with non-enzymatic cell dissociation fluid (Sigma), washed with a physiological buffer, and seeded in Cytosensor assay cups in a medium with low buffer capacity and allowed to adhere as a monolayer; alternatively, a high cell density can be achieved by using the agarose entrapment method as described in the manufacturer's protocols. The cups were then placed in the Cytosensor and equilibrated for 1 hour by gently pumping low-buffer medium over the cells. Then, low-buffer medium containing the desired amount of potential agonist was gently pumped over the cells, and the acidification rate of the cells continued to be monitored. The agonist for the expressed receptor was identified as the substance that increased the metabolic rate in transfected cells, but not the respective host cell line.

### Example 8: Screening of tissue extracts and body fluids

The microphysiometry assay of Example 7 can also be used to identify agonists in tissue extracts and body fluids. Instead of passing isolated substances over the cells as in Example 7, tissue extracts or body fluids can be used; if a response is obtained, the extract or fluid can then be fractionated using column chromatography or other separation techniques known in the art to identify, usually in multiple cycles of separation and testing of fractions, the substance which caused the effect. This way, previously unknown natural agonists for orphan receptors can be found.

### Example 9: CRE-Luciferase assay

HEK293 cells were cultured to ∼80% confluence at which time they were co-transfected with plasmids pCRE-Luc (CRE₄-Luciferase, Stratagene) and a plasmid construct containing the cDNA encoding EBI2 using the lipid transfection reagent LIPOFECTAMINE Plus⁽™⁾ (Invitrogen) as recommended by the manufacturer. Cells were cultured for 24 hours post-transfection before being washed with phosphate buffered saline (pH 7.4), recovered in Dulbecco's modified Eagle medium (supplemented with 10% (v/v) foetal calf serum) and seeded as 100µl aliquots (5×10⁴ cells) into white 96 well tissue culture plates. Following a further 24 hour incubation, compounds were added in a 20µl volume to triplet wells, and plates were left to incubate for a minimum of 5 hours. The growth media was then removed and the cells lysed in GIo lysis buffer (Promega) followed by the addition of 100µl of SteadyGlo luciferase detection reagent (Promega). The level of expressed luciferase was quantified on a Tecan Ultra⁽™⁾ Reader (1 second per well). Where experiments were designed to measure drug-mediated dose-response effects a control dose-response was always included. Data obtained for compounds were normalised relative to the control values determined in that experiment. All experiments were performed on three or more separate occasions, and mean values±S.E.M. determined. IC₅₀ values and apparent efficacies were calculated by fitting a four-parameter logistic curve (Labstats software, Microsoft Excel) through the mean normalised data. For antagonists, p*A*_{*2*} values were determined by performing Schild analysis (Arunlakshana, O. and Schild, H.O. (1959) Brit. J. Pharmacol. 14, 48-58).

### Example 10: β-lactamase assay

A CHO cell line engineered to stably contain cyclic AMP response elements (CRE) functionally linked to the coding region of reporter gene β-lactamase as well as the nuclear factor of activated T-cell promoter NF-AT (Flanagan et al (1991) Nature 352, 803-807) linked to the coding region of reporter gene β-lactamase (CHO-CRE-NFAT-BLA) is transfected stably as described in Example 4, with a plasmid containing the cDNA encoding EBI2 functionally linked to a promoter that drives expression in mammalian cells, e.g. pcDNA3.1, and selected for stable expression of EBI2.

The CHO-CRE-NFAT-BLA cells expressing EBI2 are then seeded at 4x 10³ cells per well in 96-well plates, and incubated for 60 hours at 37°C in a CO₂ incubator (5% CO₂). The medium is then removed, and 90 µl starvation medium (DMEM with high glucose, 0.1mM Non-essential amino acids, 1mM sodium pyruvate, 25mM Hepes buffer, without serum or antibiotics) is added to each well, and the cells are incubated overnight. The cells are then stimulated by addition of 10 µl urotensin I (or 1 µM ionomycin for positive control) prepared in DMEM with 1% dialysed fetal bovine serum per well. Following incubation at 37°C/5% CO₂ for 5 hours, 20µl of 6x dye solution (CCF2 Loading kit from Aurora, Cat # 00 100 012, contains solutions A-D; to prepare 6x dye solution, 36µl solution A (CCF2-AM), 180µl solution B, 2.8ml solution C and 225µl solution D are mixed according to the instructions) are added per well, and the plate is incubated on a rocking platform in the dark at room temperature for 1 hour (rocking at 40 cycles per minute). The fluorescence is then measured in a Cytofluor 4000 (PerSeptive Biosystems), using an excitation wavelength of 405 nm, and measuring emission at wavelengths of 450 nm and 530 nm.

When the ligand stimulates the receptor and the response leads to either a change in cAMP concentration or in calcium concentration in the cells, (β-lactamase will be expressed in the cells. The dye is composed of a blue (coumarin) and a green (fluorescein) component which are linked by a (β-lactam linker group. When excited at 405 nm, fluorescence energy transfer will occur within the uncleaved molecule, and the emission wavelength will be green (around 530 nm). When the linker is cleaved by β-lactamase, no energy transfer can occur, and blue fluorescence results, measured at 450 nm. Measuring the ratio of blue to green fluorescence will give an indication of receptor stimulation. The ratio is agonist dose dependent, and can be used to rank agonists for the receptor.

### Example 11: β-arrestin assay

General principle of the assay: Agonist binding to the GPCR activates G-proteins and ultimately results in second messenger cascades to elicit a physiological response. In most cases receptors occupied by agonists undergo rapid phosphorylation by GPCR Kinases (GRKs). This in turn allows β-arrestin to bind to the GPCR preventing any further interaction between the GPCR and G-protein. Internalisation of the GPCR via clathrin coated pits follows as a result of arrestin-clathrin interactions (Krupnick, J.G. and Benovic, J.L. (1998) Annual Review of Pharmacological Toxicology, 38, 289-319). By employing fusion constructs of green fluorescent protein (GFP)-β arrestin, this receptor desensitisation can be visualised using confocal microscopy. In the absence of receptor activation in a transfected cell line, fluorescence associated with the β-arrestin-GFP is evenly distributed throughout the cytoplasm. Upon receptor activation by an agonist, cytosolic β-arrestin-GFP translocates to the cell surface reflecting the interaction with the activated receptor (Evans *et al*. 2001). The use of this technology has been reported for ligand confirmation at individual receptors (Evans, N.A. (2001) Journal of Neurochemistry, 77, 476-85).

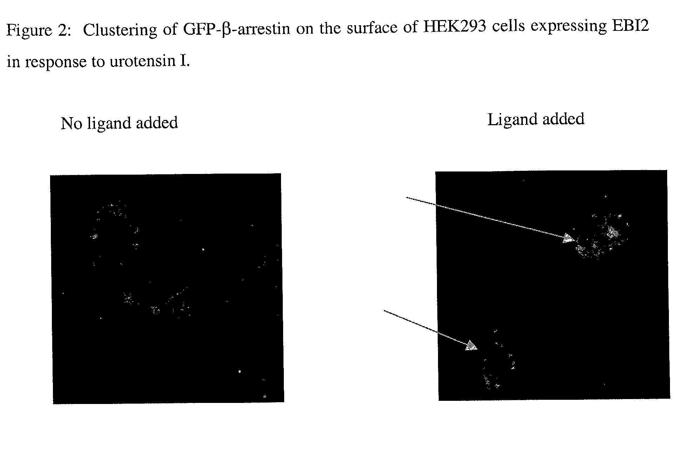

The receptor EBI2 and GFP-β-arrestin mammalian expression constructs were transiently transfected in to HEK 293 cells. Following 48hr incubation at 37°C, 5%CO2 the ligand Urotensin I was added to the cells at ∼500nM and left to incubate further for 30 minutes. The cells were then analysed using both confocal microscopy and the Cellomics ArrayScanII methodologies in order to identify internalisation of the ligand receptor GFP-β-arrestin complex. The general principals of the assay as described above indicate that any ligand able to result in internalisation of the receptor is very likely to be the functional ligand for the said receptor. In this example the experiment was designed to confirm the functional data that Urotensin I was indeed able to activate the EBI2 receptor. Figure 2 shows the confocal images without and with ligand added to the cells. Clustering of the fluorescent GFP-β-arrestin complex is clearly observed in the cells stimulated with the ligand (arrows), and clearly absent in the cells that have not been incubated with Urotensin I.

## Claims

**1.** Use of urotensin I or sauvagine or an analogue or mimetic of either one as a ligand for EBI2.

**2.** Use of urotensin I or sauvagine or an analogue or mimetic of either one as a modulator for EBI2.

**3.** Use of urotensin I or sauvagine or an analogue or mimetic of either one to elicit a functional response on EBI2.

**4.** Use of any one of claims 1 to 3, wherein the use is of urotensin I or sauvagine.

**5.** A method of screening for compounds that are modulators of EBI2, using urotensin I or sauvagine or an analogue or mimetic of either one as ligand.

**6.** A method of screening for compounds that are modulators of CRF-like receptors, including but not limited to the following steps:
(a) contacting a sample of EBI2 with a ligand selected from urotensin I or sauvagine or an analogue or mimetic of either one;
(b) contacting a similar sample of EBI2 with both the ligand used in step (a) and a test compound or mixture of test compounds; and
(c) comparing the results of (a) and (b) to determine whether the binding of the ligand used is affected by the presence of the test compound or mixture of test compounds.

**7.** A method of screening for compounds that are modulators of CRF-like receptors, including but not limited to the following steps:
(a) contacting a sample of EBI2 with a ligand, selected from urotensin I or sauvagine or an analogue or mimetic of either one, with a detectable label attached, in the presence or absence of a test compound or mixture of test compounds;
(b) measuring the amount of label bound, whereby a test compound or mixture of test compounds binding to CRF-like receptor is identified by the amount of label bound being reduced in the presence, as compared to the amount of label bound in the absence, of said test compound or mixture of test compounds.
A method of expressing CRF-like receptor, comprising transferring a suitable expression vector comprising SEQ ID NO 1 or variants or homologues thereof, into suitable host cells, and culturing said host cells under conditions suitable for the expression of the receptor.

**9.** The method of claim 8, wherein the cells are mammalian cells or insect cells.

**10.** A method of expressing CRF-like receptor, comprising upregulating the expression of EBI2 in a suitable cell.

**11.** The method of claim 6 or claim 7, wherein the sample of EBI2 comprises cells prepared by the method of any one of claims 8, 9,or 10, cells naturally expressing EBI2, or membranes prepared from said cells, or EBI2 protein enriched or purified from said cells or membranes.

**12.** A method for screening for agonists of CRF-like receptors, comprising the steps:
(a) adding a test compound or a mixture of test compounds to suitable cells expressing EBI2,
(b) measuring whether a functional response is seen.

**13.** The method of claim 12, wherein the functional response is a transient rise in intracellular calcium concentration.

**14.** The method of claim 12, wherein the functional response is acidification of the surrounding medium as measured by microphysiometry.

**15.** The method of claim 12, wherein the functional response is activation of a reporter gene linked to a cyclic AMP response element.

**16.** A method for screening for antagonists of CRF-like receptors, comprising the steps:
(a) adding a test compound or a mixture of test compounds to suitable cells expressing EBI2,
(b) adding urotensin I or sauvagine or an analogue or mimetic of either one, or an agonist as identified by the methods of any one of claims 12 to 15;
(c) measuring whether a functional response is seen, identifying antagonists as the test compound or mixture of test compounds which reduce the functional response to the agonist.

**17.** The method of any one of claims 12 to 16, wherein suitable cells expressing EBI2 are cells naturally expressing EBI2, or cells produced by the method of any one of claims 8, 9, or 10.

**18.** The compound identified by the method of any one of claims 5 to 7 or 12 to 17.

**19.** A pharmaceutical composition comprising a compound identified using the methods of any one of claims 5 to 7 or 12 to 17 and a suitable pharmaceutically acceptable carrier.

**20.** A method of preparing a pharmaceutical composition which comprises determining whether a compound is a CRF-like receptor agonist or antagonist using the method of any one of claims 5 to 7 or 12 to 17, and admixing said compound with a pharmaceutically acceptable carrier.

**21.** A method of diagnosing a CRF-like peptide-mediated disorder in a mammal, comprising
(a) measuring the level of EBI2 gene expression, or
(b) measuring the CRF-related peptide-dependent activity of EBI2 in a patient sample, and comparing said measurement to that determined from clinically normal individuals.
